# EUROPÄISCHE PATENTSCHRIFT

(11) **EP 1 746 336 B1**
(45) Veröffentlichungstag und Bekanntmachung des Hinweises auf die Patenterteilung: **21.11.2007**
(21) Anmeldenummer: 06014794.9
(22) Anmeldetag: 16.07.2006
(51) Int. Cl.: F17B 1/26, C12M 1/107

(54) **Membrane für einen Gasspeicher**
Membrane for a gas container
Membrane pour un réservoir à gaz

(30) Priorität: 21.07.2005 DE 202005011689 U
(43) Veröffentlichungstag der Anmeldung: 24.01.2007
(73) Patentinhaber: Schmack Biogas AG, 92421 Schwandorf (DE)
(72) Erfinder: Wolf, Markus, 92431 Neunburg v. W. (DE)
(74) Vertreter: Kritzenberger, Jürgen Hermann

(56) Entgegenhaltungen:
- EP-A- 0 045 114
- EP-A- 1 647 760
- WO-A-83/03884
- CH-A5- 628 130
- DE-C- 573 423
- GB-A- 1 158 664
- US-A- 1 917 622
- US-A- 2 785 825
- US-A- 4 060 175

## Beschreibung

### Technisches Gebiet

Die Erfindung betrifft einen Gasverschluss zum gasdichten Verschließen großvolumiger Behälter mittels einer Gasspeicherfolie.

### Stand der Technik

Biogasanlagen erzeugen Methan durch einen mikrobiellen Abbauprozess von organischen Substanzen. Das Biogas entsteht dabei in einem mehrstufigen Prozess, der Vergärung oder Faulung durch die Aktivität von anaeroben Mikroorganismen, d. h. unter Ausschluss von Luft. Die Art der Organismenstämme wird im Wesentlichen durch die spezifischen Prozessparameter wie Temperatur, Substrat, pH-Wert, etc. determiniert. Dadurch wird eine Anpassung der Mikroorganismen an das jeweilige Substrat erreicht, die es ermöglicht, eine Vielzahl organischer Materialien durch Fermentation abzubauen.

Das dabei entstehende Biogas kann prinzipiell einer Vielzahl von Verwertungsmöglichkeiten zugeführt werden. Bei den heute üblichen Techniken wird das Biogas meist zwischen Entstehung und Verwertung in einem großvolumigen Behälter bei gering erhöhtem Druck zwischengespeichert. Aus praktischen Gründen hat sich die Speicherung des Biogases über einem Fermentationsbehälter bzw. einem Güllelager durchgesetzt. Üblicherweise werden die Behälter, die meist aus Beton bestehen, aber zum Teil auch aus anderen Werkstoffen gefertigt sind, mit so genannten Gasspeicherfolien überspannt. Diese Folien sind robust, witterungsbeständig und in gewissem Umfang dehnbar. Gasspeicherfolien bestehen Üblicherweise aus Ethylen-Propylen-Terpolymer-Kautschuk-(EPDM-)Materialien.

Derart mit Folien überspannte Behälter werden nicht nur zur Zwischenspeicherung von Biogas, sondern auch zur Abdeckung von Gülle-Endlagem und zur Verhinderung des Ausgasens unerwünschter Stoffe aus großvolumigen Behältern in die Umgebung eingesetzt.

Zur Befestigung der Gasspeicherfolie auf der Behälterkrone werden derzeit zwei grundlegend verschiedene Technologien angewendet.

Bei der einen Befestigungsart werden Holzlatten mit trapezförmigem Querschnitt bei der Behälterherstellung in den feuchten Beton der Behälterkrone gedrückt. In die beim Abbinden des Betons entstehende trapezförmige, um den kompletten Behälter umlaufende Nut wird die Gasspeicherfolie mittels der genannten Holzlatten trapezförmigen Querschnittes eingequetscht. Nachteile dieses Verfahrens liegen in der begrenzten Witterungsbeständigkeit des Holzes, in dem hohen Aufwand bei der Herstellung / Montage des Gasverschlusses und der durch die Umstände bei der Fertigung eines hohen, großvolumigen Behälters begrenzten Genauigkeit in der Ausführung.

Die andere Befestigungsart verwendet eine auf der Behälterkrone angebrachte, komplett umlaufende Metallrinne zur Fixierung der Gasspeicherfolie. Dabei wird die Folie mittels eines mit Druckluft gefüllten Schlauches in die Rinne gequetscht. Neben dem hohen Fertigungsaufwand spricht vor allem die mangelhafte Haltekraft der Metallrinne gegen diese Art der Folienbefestigung. Hohe Windstärken vermögen die komplette Folie aus der Verankerung zu reißen. Zudem führt bereits eine minimale Beschädigung des Schlauches zum Ausfall der gesamten Befestigung. Ebenso ist ein lokal begrenzter Austausch schadhafter Abschnitte des Schlauches prinzipiell mit einem Ausfall der gesamten Befestigung verbunden.

Es besteht daher ein Bedarf an einem Gasverschluss für großvolumige Behälter mit Gasspeicherfolie, der einfach zu montieren ist und gleichzeitig einen beständigen gasdichten Verschluss des großvolumigen Behälters gewährleistet.

### Darstellung der Erfindung

Hier setzt die Erfindung an. Der Erfindung, wie sie in den Ansprüchen gekennzeichnet ist, liegt die Aufgabe zu Grunde, einen Gasverschluss für großvolumige Behälter mit Gasspeicherfolie bereitzustellen, der einfach und sicher zu montieren, unproblematisch zu öffnen und mehrfach wieder verschließbar ist und gleichzeitig einen gasdichten Verschluss des großvolumigen Behälters gewährleistet.

Diese Aufgabe wird erfindungsgemäß durch den Gasverschluss für großvolumige Behälter gemäß unabhängigem Anspruch 1 gelöst. Weitere vorteilhafte Details, Aspekte und Ausgestaltungen der Erfindung ergeben sich aus den abhängigen Ansprüchen, der Beschreibung und den Figuren.

Die vorliegende Erfindung stellt einen Gasverschluss zur Abdichtung der oberen Öffnung eines großvolumigen Behälters zur Verfügung, wobei der Behälter eine die Öffnung umlaufende Wandung aufweist. Der Gasverschluss umfasst eine in ihrem Randbereich im Bereich der Wandung aufliegende Gasspeicherfolie, zumindest vier umlaufend auf dem oberen Rand der Wandung montierte Andrückplatten und eine Dichtschnur, Der Randbereich der Gasspeicherfolie ist umgeschlagen und die Dichtschnur umlaufend in die umgeschlagene Gasspeicherfolie eingelegt. Die Andrückplatten quetschen die Gasspeicherfolie in ihrem umgeschlagenen Bereich auf den oberen Rand der Wandung.

Unter dem Begriff "großvolumiger Behälter" werden im Rahmen der vorliegenden Erfindung Behälter mit einem Innenvolumen von mindestens 10 m³ verstanden. Insbesondere fallen unter den Begriff "großvolumiger Behälter" jede Art von stationären, nicht beweglichen Behältern.

Gemäß einer bevorzugte Ausführungsform der vorliegenden Erfindung ist pro Meter Umfang der Wandung des großvolumigen Behälters zumindest eine Andrückplatte vorgesehen. Wird pro Meter Wandungsumfang eine Andrückplatte montiert, so ist ein ausreichend dichter Verschluss des großvolumigen Behälters möglich.

Besonders bevorzugt werden pro Meter Umfang der Wandung des großvolumigen Behälters zumindest zwei Andrückplatten vorgesehen. Der Abstand zwischen den einzelnen Andrückplatten ist in dieser Ausführungsform so gering, dass ein weitgehend vollständiger gasdichter Verschluss des großvolumigen Behälters erreicht wird.

Der erfindungsgemäße Gasverschluss kann zur Abdichtung der Gasspeicher von Biogasanlagen eingesetzt werden, gewährieistet aber ebenso die geruchsdichte Abdeckung von Güllelagern und sonstiger unerwünscht in die Umgebung ausgasender Großbehälter. Da bei Biogasanlagen häufig der Fermenter als Gasspeicher eingesetzt wird, dient der erfindungsgemäße Gasverschluss insbesondere dem gasdichten Verschluss von Fermentern.

Zur Montage des erfindungsgemäßen Gasverschlusses wird eine Gasspeicherfolie über den gesamten Behälterquerschnitt gelegt und auf den oberen Rand der Wandung des abzudichtenden Behälters aufgelegt. Umlaufend auf dem oberen Rand der Wandung des Behälters wird eine Dichtschnur auf die Gasspeicherfolie gelegt. Die Gasspeicherfolie wird über die Dichtschnur in Richtung des Behälterinnenraums umgeschlagen. Die Gasspeicherfolie wird dann in ihrem umgeschlagenen Bereich innerhalb der Dichtschnur mittels Andrückplatten auf die Behälterkrone gequetscht. Gerät die Gasspeicherfolie unter Zug, rutscht die Dichtschnur nach innen, bis sie am äußeren Rand der Andrückplatte zu liegen kommt und den Behälter so sicher abdichtet.

Gemäß einer bevorzugte Ausführungsform der vorliegenden Erfindung werden die Andrückplatten mittels Spannschrauben in Richtung der Behälterkrone gedrückt.

Gemäß einer weiteren bevorzugten Ausführungsform stützen sich die Andrückplatten jeweils über ein Widerlager pro Andrückplatte, bevorzugt ein Scharnier, an der Behälterkrone ab. Ist ein Widerlager vorgesehen, so kann auch bei einem geringeren Gewicht der Andrückplatten ein hoher Druck auf die Gasspeicherfolie ausgeübt werden.

Jeweils eine Andrückplatte bildet zusammen mit einer Spannschraube und einem Widerlager eine Andrückeinheit.

Gemäß einer weiteren bevorzugten Ausführungsform ist zusätzlich ein Quetschband vorgesehen, wobei die Andrückplatten Druck auf das Quetschband ausüben und die Gasspeicherfolie auf diese Weise in ihrem umgeschlagenen Bereich auf den oberen Rand der Wandung des großvolumigen Behälters quetschen. Durch das Anbringen eines Quetschbandes wird eine definierte glatte Fläche geschaffen, über die der von den Andrückplatten ausgeübte Druck auf die Gasspeicherfolie übertragen werden kann. Die Andrückplatten können in diesem Fall auch bei klein dimensionierter Auflagefläche der Andrückplatten Druck auf die Gasspeicherfolie ausüben. Durch das Quetschband wird dieser Druck auf eine größere Fläche verteilt, wodurch Beschädigungen der Folie vermieden werden. Es wird eine besonders gute Abdichtung des Behälters bei vereinfachter Montage erreicht.

Gemäß einer weiteren bevorzugten Ausführungsform ist zusätzlich eine umlaufend auf dem oberen Rand der Wandung des großvolumigen Behälters angebrachte Montageschiene vorgesehen, wobei die Gasspeicherfolie zumindest in ihrem umgeschlagenen Bereich auf der Montageschiene aufliegt. Durch das Anbringen einer Montageschiene wird eine definierte glatte Oberfläche geschaffen, auf der die Gasspeicherfolie aufliegt. Wird Druck auf die Gasspeicherfolie ausgeübt, so werden durch die glatte Auflagefläche einerseits Beschädigungen der Folie vermieden, andererseits wird eine besonders gute Abdichtung des Behälters erreicht.

Gemäß einer weiteren bevorzugten Ausführungsform der vorliegenden Erfindung weist die Montageschiene einen rechteckigen Querschnitt auf. Es handelt sich also insbesondere um ein Metallband, das die Montage des Gasverschlusses auf bereits bestehenden Behältern ermöglicht, Ältere Behälter können auf diese Weise nachträglich mit einem Gasverschluss ausgestattet werden. Ebenso können bereits vorhandene Gasverschlusssysteme durch einen Gasverschluss gemäß der vorliegenden Erfindung ersetzt werden.

Gemäß einer weiteren bevorzugten Ausführungsform der vorliegenden Erfindung weist die Montageschiene einen L-förmigen Querschnitt auf. Diese Ausführungsform ist insbesondere zur Montage auf neu zu errichtenden Behältern vorgesehen. Ein Schenkel der Montageschiene wird dabei in die Wandung des großvolumigen Behälters eingelassen,

Gemäß einer besonders bevorzugten Ausführungsform besteht die Wandung des großvolumigen Behälters aus Beton. In diesem Fall wird ein Schenkel der Montagschiene nach dem Vergießen des Betons in die Schalung der Behälterwandung in den noch feuchten Beton gedrückt Nach dem Aushärten des Betons ist die Montageschiene fest in der Wandung des Behälters verankert. Zur erleichterten Montage kann die Montageschiene mit zusätzlichen Auflageschienen ausgestattet sein, die der Auflage auf die Schalung der Behälterwandung dienen.

Gemäß einer weiteren besonders bevorzugten Ausführungsform sind auf einer Montageschiene jeweils zwei Andrückeinheiten vorgesehen. Werden zwei Andrückeinheiten pro Montageschiene angebracht, so kann einerseits der kreisrunde Querschnitt des abzudichtenden Behälters ausreichend nachgebildet werden, andererseits wird die Anzahl an einzelnen zu montierenden Einheiten möglichst gering gehalten.

Unabhängig von der Art der Follenbefestigung auf der Behälterkrone verursachen die gegenwärtig verwendeten Gasspeicherfolien Probleme aufgrund der ausgeprägten Temperaturabhängigkeit ihrer Dehnbarkeit. Unter Sonnenbestrahlung im Sommer dehnen sich die Folienspeicher selbst bei mäßiger Füllung bereits extrem aus. Hoch aufgeblähte Folienbehälter bieten dem Wind große Angriffsflächen und stellen somit eine Gefährdung des ganzen Gasspeichers dar. Die Verwendung härterer EPDM-Materialien ist aufgrund der damit verbundenen Starrheit bei tiefen Temperaturen aber nicht möglich.

Zur Lösung dieses Problems wird gemäß einer weiteren besonders bevorzugten Ausführungsform der vorliegenden Erfindung ein Netz über der Gasspeicherfolie angebracht. Dadurch wird die Dehnung der Gasspeicherfolie auch bei intensiver Sonnenbestrahlung begrenzt, da bei einer vollständigen Straffung des Netzes keine weitere Volumenvergrößerung der Gasspeicherfolie und damit des Gasspeichers möglich ist.

Gemäß einer weiteren besonders bevorzugten Ausführungsform der vorliegenden Erfindung ist das Netz an den dem großvolumigen Behälter abgewandten Enden der Andrückplatten befestigt. Mit zunehmender Füllung des Gasspeichers wird über das Netz ein Zug auf das dem Behälter abgewandte Ende der Andrückplatten ausgeübt. Über die Widerlager der Andrückplatten wird dieser Zug in einen Druck auf die Gasspeicherfolie umgesetzt. Dadurch wird mit zunehmender Füllung des Gasspeichers eine immer bessere Abdichtung des Behälters erreicht.

Eine besonders einfache Befestigung des Netzes an den dem großvolumigen Behälter abgewandten Enden der Andrückplatten ist möglich, wenn die Andrückplatten Ösen aufweisen, durch die das Netz an den Andrückplatten festgebunden werden kann.

Die im Rahmen der vorliegenden Erfindung beschriebenen Gasverschlüsse werden bevorzugt industriell vorgefertigt. Bevorzugtes Material für die aus Metall bestehenden Bestandteile der Gasverschlüsse ist ein korrosionsbeständiger, hochlegierter Stahl. Die am oberen Rand der Wandung der abzudichtenden Behälter montierten Bestandteile des Gasverschlusses sind zusammengenommen typischerweise zwischen 400 mm und 2500 mm lang und zwischen 100 mm und 500 mm breit.

### Kurze Beschreibung der Zeichnungen

Zur Illustration der Erfindung und zur Verdeutlichung ihrer Vorzüge werden nachfolgend Ausführungsbeispiele angegeben. Diese Ausführungsbeispiele sollen im Zusammenhang mit den Zeichnungen näher erläutert werden. Es versteht sich von selbst, dass diese Angaben die Erfindung nicht beschränken sollen. Es zeigen
- Fig. 1: eine bevorzugte Ausführungsform eines erfindungsgemäßen Gasverschlusses im Zustand der Montage; dargestellt ist ein Schnitt durch die Behälterwand, wobei die Schnittebene senkrecht zur Bodenfläche des Behälters liegt;
- Fig. 2: eine bevorzugte Ausführungsform eines erfindungsgemäßen Gasverschlusses im Zustand der Montage in der Aufsicht auf den oberen Rand der Wandung des Behälters;
- Fig. 3: eine bevorzugte Ausführungsform eines erfindungsgemäßen Gasverschiusses; dargestellt ist der Schnitt durch die Behälterwand gemäß Figur 1.

### Wege zur Ausführung der Erfindung

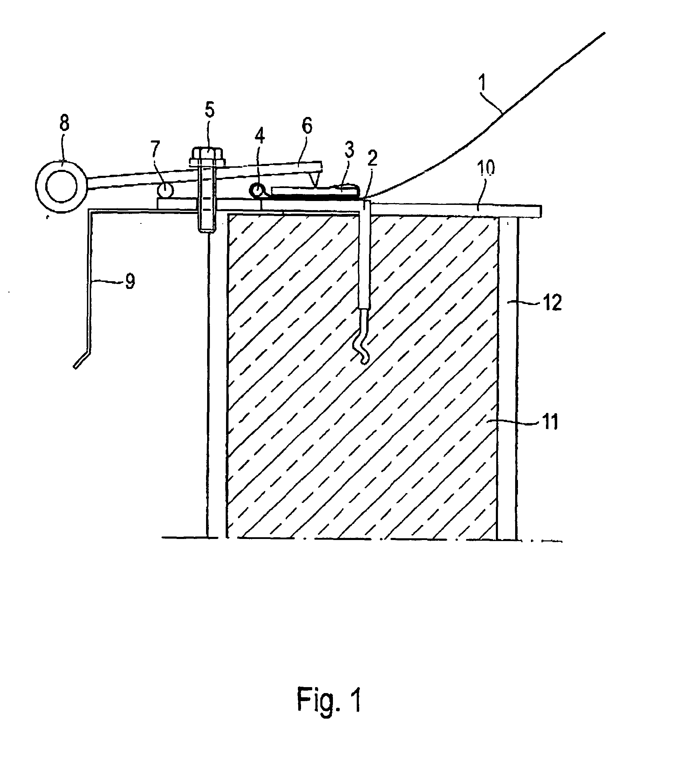

Figur 1 zeigt einen Gasverschluss im Zustand der Montage. Dargestellt ist ein Schnitt durch die Behälterwand, wobei die Schnittebene senkrecht zur Bodenfläche des Behälters liegt.

Der Gasverschluss ist zum gasdichten Verschließen großvolumiger Behälter mittels Folien konzipiert. Er besteht im Wesentlichen aus einer Gasspeicherfolie 1 und umlaufend auf der Behälterkrone zu montierenden Einheiten, in die die Gasspeicherfolie 1 eingequetscht ist. Bei der Montage wird die Gasspeicherfolie 1 außerhalb des Quetschbandes 3 nach oben umgeschlagen, in der entstehenden Falte mit einer umlaufenden 15 mm starken EPDM-Dichtschnur versehen und wieder unter dem Quetschband 3 nach innen geführt. Das Quetschband 3 wird durch Anziehen der Spannschrauben 5 mittels der Andrückplatten 6 auf die Montageschiene 2 gedrückt. Die Andrückplatte 6 ist im Scharnier 7 beweglich gelagert. Die Gasspeicherfolie 1 wird dadurch in den Einheiten mittels der Andrückplatten 6 zwischen der Montageschiene 2 und dem Quetschband 3 eingequetscht.

Das Ausführungsbeispiel beschreibt einen Gasverschluss auf einem kreisrunden Betonnachgärer mit 9 m Radius. Die Gasspeicherfolie und die Dichtschnur bestehen aus EPDM. Zum Abdichten eines Nachgärers mit 9 m Radius sind insgesamt 56 einzelne Andrückplatten vorgesehen, die umlaufend am oberen Rand der Wandung des Nachgärers montiert sind.

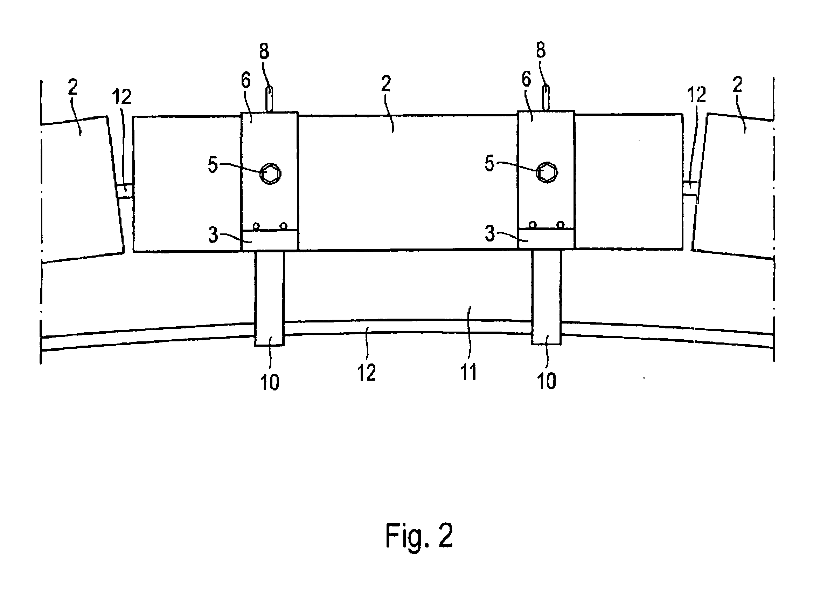

Bei der Fertigung des Betonbehälters wird zunächst die stehende Schalung 12 mit Beton gefüllt. In den feuchten Beton werden die einzelnen Andrückeinheiten eingesetzt. Das Einsetzen der Andrückeinheiten wird durch die Auflageschienen 10 erleichtert. Die Auflageschienen stützen sich auf der Behälterinnenseite auf der Schalung 12 auf. Auf der Behälteraußenseite liegen die Andrückeinheiten auf den Montageschienen 2 bzw. den darunter montierten Abschlusswinkeln 9 auf der Schalung auf (siehe Figur 2). Die Montageschienen 2 sind L-förmig ausgeführt und betonseitig mit aufgesetzten Krallen zur verbesserten Verbindung Metall/Beton versehen. Alternativ zu den Krallen können andere Formschluss bietende Details wie z. B. Löcher in den Schienen, vorgesehen sein.

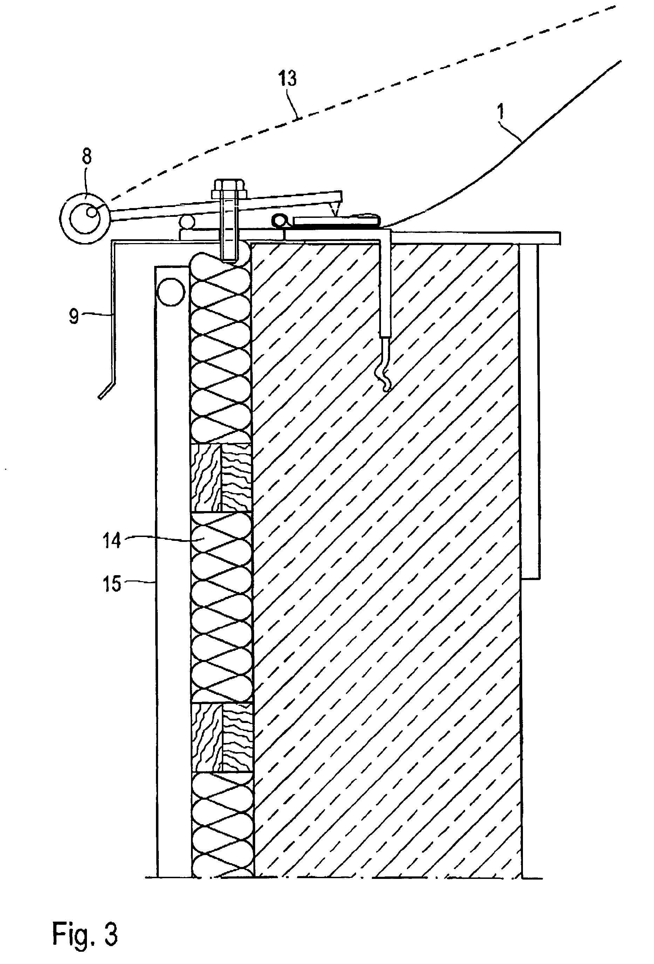

Die Andrückeinheiten können während des Behälterbaus einfach, sicher und vor allem genau auf der Behälterkrone montiert werden. Nach dem Abbinden des Betons und der Entfernung der Schalung kann der Behälter wie in Figur 3 dargestellt außen mit Dämmung 14 und Trapezblech 15 versehen werden. Der Abschlusswinkel 9 der Einheiten ist so ausgeführt, dass er Regenwasser von der Dämmung 14 abhält und zur Befestigung weiterer Bauteile wie z. B. Hinweistafeln dienen kann.

An der Außenseite der Andrückplatte befindet sich eine Öse zur Befestigung des Netzes 13. Das Netz 13 liegt über der Gasspeicherfolie und verhindert eine zu starke Ausdehnung der Gasspeicherfolie bei Erwärmung bzw. zu großem Füllgrad. Drückt die Gasspeicherfolie aufgrund hohen Füllgrades gegen das Netz, so zieht das Netz seinerseits an den Ösen 8. Der Zug an den Ösen wird über Scharnier 7 umgelenkt und bewirkt eine stärkere Quetschung der Gasspeicherfolie zwischen Quetschband 3 und Montageschiene 2.

Die Einzelteile der erfindungsgemäßen Vorrichtung bestehen, wenn nicht anders bezeichnet, aus Edelstahl. Die Montageschiene 2 ist bevorzugt als Winkel im Format 100 mm x 10 mm bei einer Länge von 990 mm mit Stahlanker ausgeführt. Bei dem Quetschband 3 handelt es sich um einen Flachstahl, dessen Querschnitt 35 mm x 8 mm und dessen Länge 990 mm beträgt. Bei den Spannschrauben 5 handelt es sich bevorzugt um M16 Schrauben mit einer Länge von 40 mm. Bei der Andrückplatte 6 schließlich handelt es sich ebenfalls bevorzugt um einen Flachstahl, dessen Querschnitt 100 mm x 10 mm und dessen Länge 150 mm beträgt und der mit Fixierdornen und Öse 8 ausgestattet ist.

### Bezugszeichenliste

- 1: Gasspeicherfolie
- 2: Montageschiene
- 3: Quetschband
- 4: Dichtschnur
- 5: Spannschraube
- 6: Andrückplatte
- 7: Scharnier
- 8: Öse
- 9: Abschlusswinkel
- 10: Auflageschiene
- 11: Behälterwand
- 12: Schalung
- 13: Netz
- 14: Dämmung
- 15: Trapezblech

## Patentansprüche

1. Gasverschluss zur Abdichtung der oberen Öffnung eines großvolumigen Behälters, wobei der Behälter eine die Öffnung umlaufende Wandung aufweist, umfassend eine in ihrem Randbereich im Bereich der Wandung aufliegende Gasspeicherfolie (1), zumindest vier umlaufend auf dem oberen Rand der Wandung montierte Andrückplatten (6) und eine Dichtschnur (4), wobei der Randbereich der Gasspeicherfolie (1) umgeschlagen ist, die Dichtschnur (4) umlaufend in die umgeschlagene Gasspeicherfolie (1) eingelegt ist und die Andrückplatten (6) die Gasspeicherfolie (1) in ihrem umgeschlagenen Bereich auf den oberen Rand der Wandung quetschen.

2. Gasverschluss nach Anspruch 1, **dadurch gekennzeichnet, dass** die Andrückplatten (6) mit Hilfe von Spannschrauben (5) die Gasspeicherfolie (1) in ihrem umgeschlagenen Bereich auf den oberen Rand der Wandung quetschen.

3. Gasverschluss nach Anspruch 1 oder 2, **dadurch gekennzeichnet, dass** zusätzlich Widerlager (7) vorgesehen sind, wobei sich die Andrückplatten (6) über die Widerlager (7) an dem oberen Rand der Wandung abstützen.

4. Gasverschluss nach einem der vorhergehenden Ansprüche, **dadurch gekennzeichnet, dass** zusätzlich ein Quetschband (3) vorgesehen ist, wobei die Andrückplatten (6) Druck auf das Quetschband (3) ausüben und die Gasspeicherfolie (1) auf diese Weise in ihrem umgeschlagenen Bereich auf den oberen Rand der Wandung quetschen.

5. Gasverschluss nach einem der vorhergehenden Ansprüche, **dadurch gekennzeichnet, dass** zusätzlich eine umlaufend auf dem oberen Rand der Wandung angebrachte Montageschiene (2) vorgesehen ist, wobei die Gasspeicherfolie (1) zumindest in ihrem umgeschlagenen Bereich auf der Montageschiene (2) aufliegt.

6. Gasverschluss nach Anspruch 5, **dadurch gekennzeichnet, dass** die Montageschiene (2) einen rechteckigen Querschnitt aufweist.

7. Gasverschluss nach Anspruch 5, **dadurch gekennzeichnet, dass** die Montageschiene (2) einen L-förmigen Querschnitt aufweist und ein Schenkel der Montageschiene in die Wandung eingelassen ist.

8. Gasverschluss nach Anspruch 7, **dadurch gekennzeichnet, dass** die Wandung des großvolumigen Behälters aus Beton besteht und ein Schenkel der Montagschiene (2) fest in dem Beton verankert ist.

9. Gasverschluss nach einem der vorhergehenden Ansprüche, **dadurch gekennzeichnet, dass** ein Netz (13) über der Gasspeicherfolie (1) angebracht ist.

10. Gasverschluss nach Anspruch 9, **dadurch gekennzeichnet, dass** das Netz (13) an den dem großvolumigen Behälter abgewandten Enden der Andrückplatten (6) befestigt ist.

11. Gasverschluss nach Anspruch 10, **dadurch gekennzeichnet, dass** das dem großvolumigen Behälter abgewandte Ende der Andrückplatte (6) als Öse (8) ausgestaltet ist.

12. Gasverschluss nach einem der vorhergehenden Ansprüche, **dadurch gekennzeichnet, dass** pro Meter Umfang der Wandung des großvolumigen Behälters zumindest eine Andrückplatte (6) vorgesehen ist.

13. Gasverschluss nach einem der vorhergehenden Ansprüche, **dadurch gekennzeichnet, dass** pro Meter Umfang der Wandung des großvolumigen Behälters zumindest zwei Andrückplatten (6) vorgesehen sind.

14. Fermenter einer Biogasanlage mit zumindest einem Gasverschluss wie in den vorhergehenden Ansprüchen definiert.

## Claims

1. A gas seal for sealing the upper opening of a large-volume container, the container exhibiting a wall encircling the opening, comprising a gas holder foil (1) resting in its peripheral area in the area of the wall, at least four pinch plates (6) mounted circumferentially on the upper edge of the wall, and a sealing cord (4), the peripheral area of the gas holder foil (1) being overturned, the sealing cord (4) being inserted circumferentially in the overturned gas holder foil (1), and the pinch plates (6) squeezing the gas holder foil (1) in its overturned area to the upper edge of the wall.

2. The gas seal according to claim 1, **characterized in that** the pinch plates (6) squeeze the gas holder foil (1) in its overturned area to the upper edge of the wall with the aid of set screws (5).

3. The gas seal according to claim 1 or 2, **characterized in that**, additionally, counter bearings (7) are provided, the pinch plates (6) being supported on the upper edge of the wall via the counter bearings (7).

4. The gas seal according to one of the preceding claims, **characterized in that**, additionally, a squish band (3) is provide, the pinch plates (6) exerting pressure on the squish band (3) and the gas holder foil (1) in this way being squeezed in its overturned area to the upper edge of the wall.

5. The gas seal according to one of the preceding claims, **characterized in that**, additionally, a mounting rail (2) attached circumferentially to the upper edge of the wall is provided, the gas holder foil (1) resting at least in its overturned area on the mounting rail (2).

6. The gas seal according to claim 5, **characterized in that** the mounting rail (2) exhibits a rectangular cross section.

7. The gas seal according to claim 5, **characterized in that** the mounting rail (2) exhibits an L-shaped cross section and one leg of the mounting rail is recessed into the wall.

8. The gas seal according to claim 7, **characterized in that** the wall of the large-volume container consists of concrete and one leg of the mounting rail (2) is anchored firmly in the concrete.

9. The gas seal according to one of the preceding claims, **characterized in that** a net (13) is attached over the gas holder foil (1).

10. The gas seal according to claim 9, **characterized in that** the net (13) is fastened to the end of the pinch plates (6) facing away from the large-volume container.

11. The gas seal according to claim 10, **characterized in that** the end of the pinch plate (6) facing away from the large-volume container is designed as an eyelet (8).

12. The gas seal according to one of the preceding claims, **characterized in that** at least one pinch plate (6) is provided per meter perimeter of the wall of the large-volume container.

13. The gas seal according to one of the preceding claims, **characterized in that** at least two pinch plates (6) are provided per meter perimeter of the wall of the large-volume container.

14. A fermenter of a biogas plant having at least one gas seal as defined in the preceding claims.

## Revendications

1. Obturateur pour gaz pour rendre étanche l'ouverture supérieure d'un réservoir de grand volume, le réservoir présentant une paroi entourant l'ouverture, comprenant une feuille d'accumulateur de gaz (1) reposant dans sa zone de bordure, dans la région de la paroi, au moins quatre plaques de pression (6) montées périphériquement sur le bord supérieur de la paroi, et un cordon d'étanchéité (4), la zone de bordure de la feuille d'accumulateur de gaz (1) étant rabattue, le cordon d'étanchéité (4) étant placé périphériquement dans la feuille d'accumulateur de gaz (1) rabattue et les plaques de pression (6) écrasant la feuille d'accumulateur de gaz (1) dans sa zone rabattue, sur le bord supérieur de la paroi.

2. Obturateur pour gaz selon la revendication 1, **caractérisé en ce que** les plaques de pression (6) écrasent, à l'aide de vis de serrage (5), la feuille d'accumulateur de gaz (1), dans sa zone rabattue, sur le bord supérieur de la paroi.

3. Obturateur pour gaz selon la revendication 1 ou 2, **caractérisé en ce que** sont prévues en outre des butées (7), les plaques de pression (6) prenant appui sur le bord supérieur de la paroi, par l'intermédiaire des butées (7).

4. Obturateur pour gaz selon l'une des revendications précédentes, **caractérisé en ce qu'**il est prévu en outre un ruban d'écrasement (3), les plaques de pression (6) exerçant une pression sur le ruban d'écrasement (3) et écrasant la feuille d'accumulateur de gaz (1) de cette façon, dans sa zone rabattue, sur le bord supérieur de la paroi.

5. Obturateur pour gaz selon l'une des revendications précédentes, **caractérisé en ce qu'**il est prévu en outre un rail de montage (2) placé périphériquement sur le bord supérieur de la paroi, la feuille d'accumulateur de gaz (1) reposant au moins dans sa zone rabattue, sur le rail de montage (2).

6. Obturateur pour gaz selon la revendication 5, **caractérisé en ce que** le rail de montage (2) présente une section transversale rectangulaire.

7. Obturateur pour gaz selon la revendication 5, **caractérisé en ce que** le rail de montage (2) présente une section transversale en L et une aile du rail de montage est encastrée dans la paroi.

8. Obturateur pour gaz selon la revendication 7, **caractérisé en ce que** la paroi du récipient de grand volume est en béton et une aile du rail de montage (2) est ancrée de manière fixe dans le béton.

9. Obturateur pour gaz selon l'une des revendications précédentes, **caractérisé en ce qu'**un filet (13) est placé sur la feuille d'accumulateur de gaz (1).

10. Obturateur pour gaz selon la revendication 9, **caractérisé en ce que** le filet (13) est fixé aux extrémités, tournées à l'opposé du réservoir de grand volume, des plaques de pression (6).

11. Obturateur pour gaz selon la revendication 10, **caractérisé en ce que** l'extrémité, tournée à l'opposé du réservoir de grand volume, de la plaque de pression (6), est réalisée sous la forme d'un oeillet (8).

12. Obturateur pour gaz selon l'une des revendications précédentes, **caractérisé en ce qu'**il est prévu au moins une plaque de pression (6) par mètre de circonférence de la paroi du réservoir de grand volume.

13. Obturateur pour gaz selon l'une des revendications précédentes, **caractérisé en ce que** sont prévues au moins deux plaques de pression (6) par mètre de circonférence de la paroi du réservoir de grand volume.

14. Cuve de fermentation d'une installation de biogaz avec au moins une obturateur pour gaz comme défini dans les revendications précédentes.
